# EUROPEAN PATENT APPLICATION

(11) **EP 4 763 148 A1**
(43) Date of publication of application: **24.06.2026**
(21) Application number: 24855662.3
(22) Date of filing: 13.08.2024
(51) Int. Cl.: A61F 2/848, A61B 17/34, A61F 2/24

(54) **IMPLANT**

(30) Priority: 18.08.2023 CN 202311053444
(71) Applicant: United Innomed (Shanghai) Limited, Shanghai 201315 (CN)
(72) Inventor: GE, Shuchen, Shanghai 201315 (CN); WANG, Li, Shanghai 201315 (CN); LI, Bo, Shanghai 201315 (CN)
(74) Representative: Murgitroyd & Company
(86) International application number: PCT/CN2024/111805
(87) International publication number: WO 2025/039936

(57) **Abstract**

An implant includes a main body (100) and at least one clamping member group (200). The main body (100) is configured to be fixed to a target part and has a predetermined length. The clamping member group (200) includes a first clamping member (210) and a second clamping member (220), and the first clamping member (210) and the second clamping member (220) are distributed on a periphery of the main body (100) at an interval along the axial direction of the main body (100). The first clamping member (210) and the second clamping member (220) are configured to be elastically deformable.

## Description

### TECHNICAL FIELD

The present disclosure relates to the field of interventional medical devices, in particular, to an implant.

### BACKGROUND

Cardiac or vascular implants can be used to improve the physiological function of a target part, monitor physiological parameters of the target part, or perform both of the aforementioned functions. The implant needs to remain in the target part to function properly. For example, heart failure is a clinical syndrome caused by abnormal ventricular filling and ejection functions. To prevent an increase in left atrial pressure, a monitoring device can be implanted in the interatrial septum to continuously monitor left atrial pressure. When persistent pressure elevation occurs, a shunt device can also be implanted to partially divert blood from the left atrium to the right atrium, thereby reducing left atrial pressure. Alternatively, a device that integrates both monitoring and shunting functions can be directly implanted to perform both tasks simultaneously.

In the prior art, clamping members are used to clamp the tissue at the target part, thereby fixing the shunt device or sensor to the bodily tissue, for example, to the interatrial septum. However, existing designs of clamping members have numerous shortcomings. For instance, in a mesh-disc type shunt device, the clamping members are disc-shaped and parallel to each other. The portions of the discs that engage with the tissue are difficult or unable to adaptively adjust according to the thickness or shape of the tissue. This may result in insufficient clamping strength or excessive clamping that damages the tissue. Additionally, when the clamped tissue is relatively thick or when surrounding tissues are present, disc-shaped clamping members may deform to some extent and compress the shunt structure between the two clamping members, leading to a reduction in the aperture of the shunt channel and impairing shunt performance.

### SUMMARY

In view of this, the present disclosure provides an implant adaptable to more target positions.

On the first aspect, an implant is provided for improving the physiological function and/or detecting physiological indicators of the target part. The implant includes a main body and at least one clamping member group. The main body is configured to be fixed to the target part and has a predetermined length. The clamping member group includes a first clamping member and a second clamping member. The first clamping member and the second clamping member are distributed on a periphery of the main body at intervals along the axial direction of the main body. The first clamping member and the second clamping member are configured to be elastically deformable so as to move away from each other along the axial direction of the main body and/or to contract along the radial direction of the main body.

In a possible implementation, the first clamping member and/or the second clamping member includes a connecting segment and a clamping segment. One end of the connecting segment is connected to the main body and the other end is connected to the clamping segment. The first clamping member and the second clamping member protrude entirely from the outer peripheral surface of the main body. The clamping segment and/or the connecting segment is configured to be deformable so as to move away from each other along the axial direction of the main body and/or to contract along the radial direction of the main body.

In combination with the above possible implementation, in another possible implementation, the clamping segment and/or the connecting segment deforms in a form of circumferential deflection and/or bending toward the main body along the radial direction so as to contract along the radial direction of the main body.

In combination with the above possible implementation, in another possible implementation, a bend portion with a variable angle is provided between the clamping segment and the connecting segment, so that the clamping segment is capable of being compressed along the radial direction of the main body.

In combination with the above possible implementation, in another possible implementation, the clamping segment is configured such that at least a part thereof is capable of changing the angle between the clamping segment and the connecting segment in a form of circumferential deflection and/or bending toward the main body, so that the clamping segment is capable of being compressed along the radial direction of the main body.

In combination with the above possible implementation, in another possible implementation, the outermost end of the clamping segment is a smooth structure.

In combination with the above possible implementation, in another possible implementation, the clamping segment is formed by bending or winding an elastic wire or an elastic sheet, and the end of the elastic wire or the elastic sheet is located between the outermost end and the main body; or, the clamping segment is formed by cutting an elastic sheet, and the end of the elastic sheet is arc-shaped.

In combination with the above possible implementation, in another possible implementation, when the clamping segment is formed by winding the elastic wire or the elastic sheet, the end of the elastic wire or the elastic sheet is located inside a wound structure.

In combination with the above possible implementation, in another possible implementation, the elastic wire or the elastic sheet is wound or the end of the elastic wire or the elastic sheet is wound so that the outermost end of the clamping segment is a smooth structure.

In combination with the above possible implementation, in another possible implementation, the first clamping member and the second clamping member further bend circumferentially to be capable of deflecting circumferentially relative to the main body under an external force, the first clamping member and the second clamping member form an angle with the main body, and the angle is less than 90 degrees; and/or, the circumferential width of the connecting segment of the first clamping member and the second clamping member is less than the width of the clamping segment and/or the width of the bend portion, so as to facilitate the first clamping member and the second clamping member to deflect circumferentially relative to the main body under an external force.

In combination with the above possible implementation, in another possible implementation, the clamping segment includes a clamping portion, and the clamping portion of the first clamping member and the clamping portion of the second clamping member have a minimum spacing between the first clamping member and the second clamping member along the axial direction of the main body; and/or, the clamping portion of the first clamping member and the clamping portion of the second clamping member are parallel to each other.

In combination with the above possible implementation, in another possible implementation, the clamping portion is a ring structure in an axial or radial view of the main body.

In combination with the above possible implementation, in another possible implementation, the ring structure is formed by winding a metal wire.

In combination with the above possible implementation, in another possible implementation, the metal wire is wound in an interlaced form in the ring structure.

In combination with the above possible implementation, in another possible implementation, the number of the clamping member groups is multiple, and the clamping portions of at least two clamping member groups are located at different axial positions relative to the main body.

In combination with the above possible implementation, in another possible implementation, the first clamping member and the second clamping member are different in shape, so that the clamping portion is offset to one end of the main body.

In combination with the above possible implementation, in another possible implementation, the first clamping member and the second clamping member are partially offset or completely offset along the circumferential direction.

In combination with the above possible implementation, in another possible implementation, the first clamping member and the second clamping member are partially offset along the circumferential direction, with their starting ends offset circumferentially and their ends coinciding.

In combination with the above possible implementation, in another possible implementation, the clamping segment includes a clamping portion, and the clamping portion is provided with anti-skid protrusions or anti-skid holes.

In combination with the above possible implementation, in another possible implementation, the outermost end is a ring structure, and the ring structure is provided with a radiopaque material.

In combination with the above possible implementation, in another possible implementation, the main body includes a cylinder, the cylinder is formed by spirally winding an elastic curved segment made of metal material, and the first clamping member and the second clamping member are integrally formed with the cylinder.

In combination with the above possible implementation, in another possible implementation, the main body includes a cylinder of a shunt device; or includes a sensor and a connecting member, and the connecting member is connected to the clamping member group.

According to the implant provided in the present disclosure, the clamping member is configured to be deformable along the radial direction to accommodate different tissue thicknesses at the target part, or to be deformable along the circumferential direction to adapt to different spatial structures at the target part, thereby reducing damage to the tissues of the target part.

### BRIEF DESCRIPTION OF THE DRAWINGS

For a clearer description of the technical solutions in the embodiments of the present disclosure, a brief introduction to the drawings required in the embodiments will be provided below.

It should be understood that the following drawings merely illustrate certain embodiments of the present disclosure and should not be construed as limiting the scope.

It should also be understood that the same or similar reference numerals are used in the drawings to denote the same or similar elements.

It should also be understood that the drawings are schematic, and the dimensions and proportions of the elements in the drawings may not be precise.
FIG. 1a is a schematic perspective view of an implant according to one embodiment of the present disclosure.
FIG. 1b is a side view of the implant in Figure 1a.
FIG. 2 is a schematic structural view of an implant according to another embodiment of the present disclosure.
FIG. 3 is a schematic structural view of an implant according to another embodiment of the present disclosure.
FIG. 4 is a schematic structural view of an implant according to another embodiment of the present disclosure.
FIG. 5 is a schematic structural view of an implant according to another embodiment of the present disclosure.
FIG. 6 is a schematic structural view of an implant according to another embodiment of the present disclosure.
FIG. 7 is a schematic structural view of an implant according to another embodiment of the present disclosure.
FIG. 8 is a schematic view of the implant when placed between the left atrium and the coronary sinus.
FIG. 9 is a schematic structural view of an implant according to another embodiment of the present disclosure.
FIG. 10 is a schematic structural view of an implant according to another embodiment of the present disclosure.
FIG. 11 is a schematic structural view of an implant according to another embodiment of the present disclosure.
FIG. 12 is a schematic structural view of an implant according to another embodiment of the present disclosure.
FIG. 13 is a schematic structural view of an implant according to another embodiment of the present disclosure.
FIG. 14 is a schematic structural view of an implant according to another embodiment of the present disclosure.
FIG. 15 is a schematic structural view of an implant according to another embodiment of the present disclosure.
FIG. 16 is a schematic structural view of an implant according to another embodiment of the present disclosure.
FIG. 17 is a schematic structural view of an implant according to another embodiment of the present disclosure.
FIG. 18 is a schematic structural view of an implant according to another embodiment of the present disclosure.
FIG. 19 is a schematic structural view of an implant according to another embodiment of the present disclosure.
FIG. 20 is a schematic structural view of an implant according to another embodiment of the present disclosure.
FIG. 21 is a schematic view of the winding manner of the clamping member in Figure 20.
FIG. 22 is a schematic view of the winding manner of the clamping member in an alternative embodiment.
FIG. 23 is a schematic structural view of an implant according to another embodiment of the present disclosure.
FIG. 24 is a schematic structural view of an implant according to another embodiment of the present disclosure.
FIG. 25 is a schematic structural view of an implant according to another embodiment of the present disclosure.

### DETAILED DESCRIPTIONS OF THE EMBODIMENTS

Hereinafter, exemplary embodiments of the present disclosure will be described with reference to the accompanying drawings. It should be understood that the present disclosure may be implemented in various ways and should not be construed as being limited to the embodiments set forth herein. The embodiments described herein are provided only to facilitate a more thorough and complete understanding of the present disclosure.

It should be understood that the term "include" and its variations used in the present disclosure are open-ended inclusions, meaning "including but not limited to". The term "according to" means "at least partially based on", and the term "a plurality of" means "two or more".

It should be understood that although terms such as "first" or "second" may be used in the present disclosure to describe various elements, these elements are not limited by such terms. These terms are used merely to distinguish one element from another.

The present disclosure provides an implant for improving physiological functions and/or detecting physiological indicators of a target part. The implant includes a main body and at least one clamping member group. The main body is configured to be fixed to the target part and has a predetermined length. The clamping member group includes a first clamping member and a second clamping member, and the first clamping member and the second clamping member are distributed on the periphery of the main body at an interval along the length direction (i.e., the axial direction) of the main body. The clamping member group is configured such that the first clamping member and the second clamping member are elastically deformable to change the spacing along the axial direction of the main body and/or contract along the radial direction of the main body. When the thickness of the target part is greater than the spacing between the first clamping member and the second clamping member, the first clamping member and the second clamping member can change the spacing along the axial direction of the main body and/or contract along the radial direction of the main body to adaptively conform to the morphology of the tissue at the target part, thereby reducing damage to the tissue.

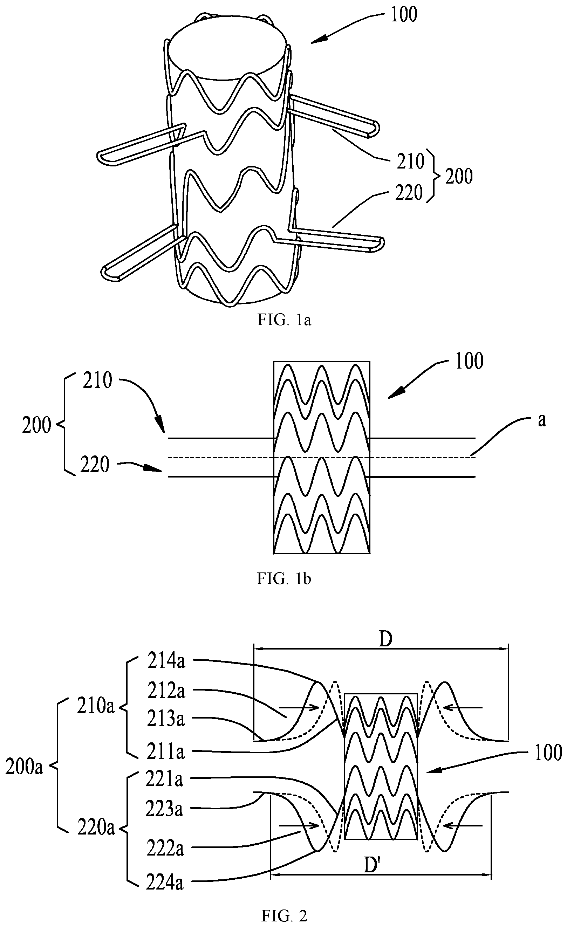

Referring to FIG. 1a and FIG. 1b, FIG. 1a is a schematic perspective view of an implant according to an embodiment of the present disclosure, and FIG. 1b is a side view of the implant in FIG. 1a.

In this embodiment, the implant is a shunt device, including a main body 100 and a plurality of clamping member groups 200. The main body 100 is a cylinder of the shunt device, the cylinder is formed by weaving elastic wires, and the inside and/or outside of the cylinder is provided with a covering film. Each clamping member group 200 includes a first clamping member 210 and a second clamping member 220. The first clamping member 210 and the second clamping member 220 in the same clamping member group are distributed along the axial direction of the main body 100, with a predetermined distance between them. The first clamping member 210 and the second clamping member 220 are rod-shaped structures, and perpendicularly connected to the surface of the main body 100. In this embodiment, the first clamping member 210 and the second clamping member 220 are integrally formed with the elastic wires constituting the main body 100. In this embodiment, the first clamping members 210 in each group have the same axial position relative to the main body 100 and are uniformly distributed around the outer peripheral surface of the main body 100 about the central axis. The second clamping members 220 in each group have the same axial position relative to the main body 100 and are uniformly distributed around the outer peripheral surface of the main body 100 about the central axis. The plurality of first clamping members 210 define one positioning structure along the axial direction of the main body 100, and the plurality of second clamping members 220 define another positioning structure. When the main body 100 is inserted into a puncture/foramen ovale in the interatrial septum, the two positioning structures are distributed on both sides of the interatrial septum to clamp it, thereby keeping the main body 100 in the puncture/foramen ovale. In FIG. 1b, the dashed line a indicates the orientation of the interatrial septum, which is parallel to the first clamping member 210 or the second clamping member 220, i.e., perpendicular to the main body 100.

The first clamping member 210 and the second clamping member 220 are rod-shaped structures. Compared to disc-shaped clamping structures, when the thickness of the interatrial septum is greater than the spacing between the first clamping member 210 and the second clamping member 220, the two can deform and move away from each other under the pressure of the interatrial septum, thereby adapting to the tissue thickness without compressing the main body 100 and affecting the channel size of the shunt region in the main body 100. In this way, the clamping member groups 200 can both reduce pressure on the tissue to some extent, thereby minimizing damage to the tissue, and maintain the original shunting capability.

Furthermore, the cylinder of the main body 100 is formed by spirally winding an elastic curved segment made of metal material. Specifically, the elastic curved segment extends circumferentially in an annular manner for one turn from a starting position, then extends upward in a spiral manner for one or more turns, and then extends circumferentially in an annular manner for one full turn, thereby forming the cylinder. The elastic curved segment is a wavy-shaped metal segment. Compared to mesh-woven or cut structures, this cylinder structure allows the parts where the first clamping member 210 or the second clamping member 220 connects to the cylinder to have a higher degree of freedom, without being constrained by other structures on the cylinder, making it easier for the first clamping member 210 or the second clamping member 220 to change their relative positions with the cylinder under external forces. Preferably, the first clamping member 210 and the second clamping member 220 are also formed by winding the elastic curved segment. Specifically, a part of the elastic curved segment extends radially outward from the surface of the cylinder and then folds back to the surface of the cylinder to form the first clamping member 210 or the second clamping member 220. Compared to manners where the first clamping member 210 and the second clamping member 220 are fixed to the cylinder through processes such as welding or bonding, this approach provides higher connection strength between the first clamping member 210, the second clamping member 220 and the cylinder, ensuring that the first clamping member 210 and the second clamping member 220 do not detach from the cylinder due to positional changes.

In other embodiments, the cylinder of the main body 100 can also be a mesh-woven or cut structure, and the first clamping member 210 and the second clamping member 220 can also be fixed to the cylinder through processes such as welding or bonding. The present disclosure does not impose limitations in this regard.

Referring to FIG. 2, FIG. 2 is a schematic structural diagram of an implant according to another embodiment of the present disclosure. In this embodiment, the implant includes a main body 100 and a clamping member group 200a. The clamping member group 200a includes a first clamping member 210a and a second clamping member 220a. Other similarities between this embodiment and the previous one will not be repeated here. The difference from the previous embodiment is that the first clamping member 210a and the second clamping member 220a in this embodiment can deform along the radial direction of the main body 100, thereby reducing the outer diameter of the implant. Specifically, both the first clamping member 210a and the second clamping member 220a protrude entirely from the outer peripheral surface of the main body 100, and they achieve contraction along the radial direction of the main body 100 through partial structures bending and deforming in the radial direction toward the main body 100.

The first clamping member 210a includes a first connecting segment 211a, a first clamping segment 212a, a first clamping portion 213a, and a first bend portion 214a. One end of the first connecting segment 211a is connected to the main body 100, and the other end is connected to the first clamping segment 212a. When viewed radially, they form a roughly inverted V-shaped structure, with their junction being the first bend portion 214a. The end segment of the first clamping segment 212a extends approximately radially outward from the main body 100 to form the first clamping portion 213a. In other words, as the first clamping member 210a extends radially outward, its shape relative to a horizontal reference appears as "low-high-low". The horizontal reference may be the lower end surface of the main body 100. The second clamping member 220a includes a second connecting segment 221a, a second clamping segment 222a, a second clamping portion 223a, and a second bend portion 224a. The components of the second clamping member 220a are in planar symmetry with those of the first clamping member 210a, and the symmetry plane is the cross-section through the axial midpoint of the main body 100.

The dashed lines in FIG. 2 represent the deformed shapes of the first clamping member 210 and the second clamping member 220 when subjected to radial forces. Due to the presence of the first bend portion 214a and the second bend portion 224a, the first clamping segment 212a can move closer to the first connecting segment 211a, and the first connecting segment 211a can move closer to the main body 100, the second clamping segment 222a can move closer to the second connecting segment 221a, and the second connecting segment 221a can move closer to the main body 100. This effectively compresses the first clamping member 210a and the second clamping member 220a radially. After compression, the maximum diameter D' of the circumscribed circle at the outermost side of the first clamping member 210a and the second clamping member 220a is less than the maximum diameter D of the circumscribed circle before compression. In this way, when there is abundant tissue around the main body 100, the surrounding tissue can compress the first clamping member 210a and the second clamping member 220a radially to reduce damage to the tissue.

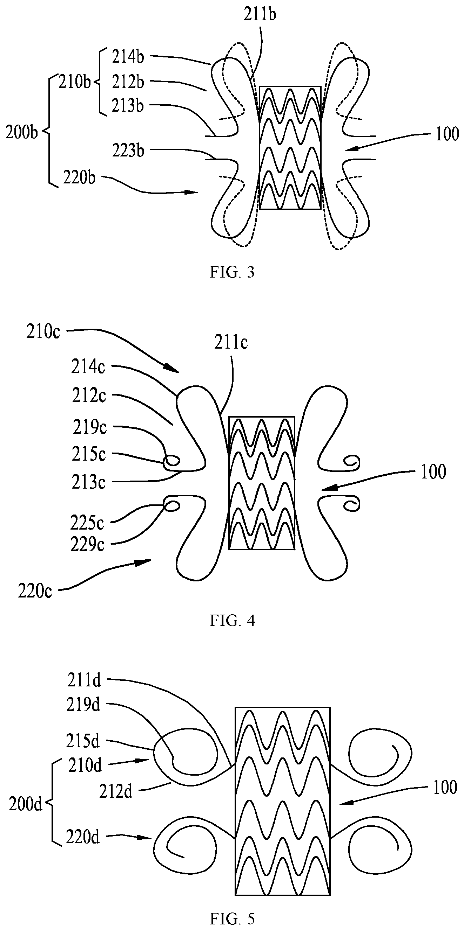

Referring to FIG. 3, FIG. 3 is a schematic structural diagram of an implant according to another embodiment of the present disclosure. In this embodiment, the implant includes a main body 100 and a clamping member group 200b. The clamping member group 200b includes a first clamping member 210b and a second clamping member 220b. Other similarities between this embodiment and the previous one will not be repeated here. The difference from the previous embodiment is that the first clamping member 210b and the second clamping member 220b in this embodiment can not only deform along the radial direction of the main body 100 to reduce the outer diameter of the implant but also move away from each other along the axial direction, further avoiding tissue damage. Specifically, the first clamping member 210b includes a first connecting segment 211b, a first clamping segment 212b, a first clamping portion 213b, and a first bend portion 214b. One end of the first connecting segment 211b is connected to the main body 100, and the other end is connected to the first clamping segment 212b. When viewed radially, they appear roughly S-shaped, with their junction being the first bend portion 214b. The end segment of the first clamping segment 212b extends approximately radially outward from the main body 100 to form the first clamping portion 213b. Thus, as the first clamping member 210b extends radially outward, its shape relative to a horizontal reference (a horizontal plane perpendicular to the axis between the first clamping member 210 and the second clamping member 220) appears as "low-high-low" and relative to a vertical reference (the axis of the main body 100) appears as "near-far-near". This configuration effectively means that the first clamping segment 212b does not extend further outward but instead folds back and bends toward the main body 100. The horizontal reference may be the lower end surface of the main body 100. The vertical reference may be the centerline of the main body 100. The components of the second clamping member 220b are in planar symmetry with those of the first clamping member 210b, and the symmetry plane is the cross-section at the midpoint of the main body 100. In this embodiment, the first bend portion 214b is a curved segment with a large radius, allowing a larger spacing and a greater range of angular variation between the first clamping segment 212b and the first connecting segment 211b. Thus, the first clamping segment 212b can move closer to the main body 100 along the radial direction, and also move along the axial direction (as shown by the dashed structure in FIG. 3). When there is abundant tissue around the target part of the implant, the surrounding tissue can drive the first clamping member 210b and the second clamping member 220b to contract along the radial direction from both sides, avoiding tissue damage. Similarly, when the tissue at the target location is thicker, it can drive the upper and lower clamping members to move along the axial direction away from each other, also avoiding tissue damage.

The first clamping portion 213b is the part at the free end of the first clamping member 210b, which has a certain length and is parallel to the end surface of the main body 100. Additionally, the first clamping portion 213b of the first clamping member 210b is parallel to the second clamping portion 223b of the second clamping member 220b. The first clamping portion 213b and the second clamping portion 223b are the parts where the first clamping member 210b and the second clamping member 220b have a minimum spacing along the axial direction of the main body 100, so that they are the primary regions for forming the clamping action. In this way, when the first clamping segment 212b move along the axial direction, the first clamping portion 213b and the second clamping portion 223b can still form surface/line contact with the tissue. This configuration can create a larger clamping area, resulting in a more stable clamping effect.

It is understandable that in other embodiments, the first clamping portion 213a located at the end segment of the first clamping segment 212a and the second clamping portion 223a located at the end of the second clamping segment 222a may not necessarily extend outward along the radial direction of the main body 100, but extend toward the clamped tissue. When the first clamping member 210 and the second clamping member 220 are formed by winding an elastic curved segment (such as a U-shaped structure wound from a metal wire), the relative positions of the first clamping portion 213a and the second clamping portion 223a can be flexibly adjusted according to the thickness of the tissue. Their ends are smooth and will not damage the tissue. In other embodiments, the first clamping member 210 and the second clamping member 220 may also be sheet-like structures rather than wound from an elastic curved segment, and ends of the first clamping member 210 and the second clamping member 220 can be smoothed to prevent tissue damage.

Referring to FIG. 4, FIG. 4 is a schematic structural diagram of an implant according to another embodiment of the present disclosure. The implant in this embodiment has a structure substantially identical to that of the implant in FIG. 3, with the difference being that the free ends of the first clamping member 210c and the second clamping member 220c are wound structures. This makes the outermost side 215c of the first clamping member 210c and the outermost side 225c of the second clamping member 220c smooth structures, meaning they have smooth contours. The end 219c of the first clamping member 210c and the end 229c of the second clamping member 220c are located at the center of the wound structure (i.e., between the outermost side of the clamping member and the main body 100) and will not puncture surrounding tissue. In this case, the clamping member may be a flat metal sheet without smoothed ends, or, combined with the above embodiment, it may be a structure wound from a single metal wire, appearing roughly U-shaped when viewed from above or below.

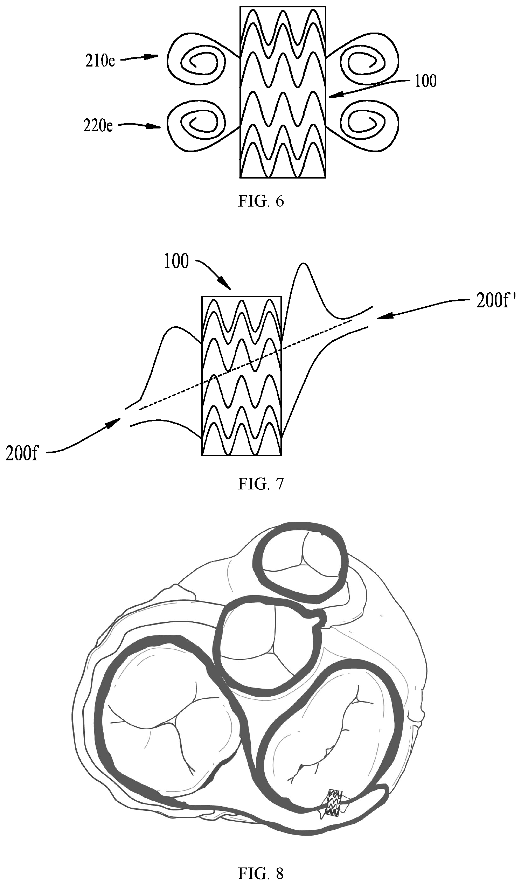

Referring to FIG. 5 and FIG. 6, two implants with different clamping members are shown. FIG. 5 is a schematic structural diagram of an implant according to another embodiment of the present disclosure. The implant includes a main body 100 and a clamping member group 200d. The clamping member group 200d includes a first clamping member 210d and a second clamping member 220d. The first clamping member 210d includes a first connecting segment 211d and a first clamping segment 212d. Other similarities between this embodiment and the previous one will not be repeated here. The difference is that the first clamping segment 212d is entirely a wound structure. In the first clamping member 210d, the first clamping segment 212d is wound outward along the axial direction, i.e., upward in the figure. Additionally, the free end is a wound structure, making the outermost side 215d of the first clamping member 210d a smooth structure with a rounded contour. The end 219d of the first clamping member 210d is located at the center of the wound structure (i.e., between the outermost side of the clamping member and the main body 100) and will not puncture surrounding tissue. The second clamping member 220d is in planar symmetry with the first clamping member 210d, and the symmetry plane is the cross-section through the axial midpoint of the main body 100.

When a clamping member has at least one curved portion in both the axial direction and the radial direction, it can provide a certain degree of elasticity in both directions, making it suitable for adjusting the overall radial dimension of the implant and the spacing between the upper and lower clamping members based on the implantation environment. In the first clamping member 210d, the first clamping segment 212d is entirely a wound structure, meaning it has curved portions in all directions. The greater the number of winding turns, the smaller the degree of radial deformability of the wound portion. Therefore, by adjusting the number of turns, the desired radial dimensional adjustability and the ideal post-deformation shape can be achieved.

In some optional embodiments, the member of turns wound of the clamping segment of the implant may be more, for example, more than one or more additional turns than the first clamping segment 212d in FIG. 5.

FIG. 6 is a schematic structural diagram of an implant according to another embodiment of the present disclosure. Compared with the implant shown in FIG. 5, the winding direction of the clamping segments is opposite. That is, the clamping segments of the first clamping member 210e and the second clamping member 220e are both wound inward, i.e., wound toward each other.

Referring to FIG. 7 and FIG. 8, FIG. 7 is a schematic structural diagram of an implant according to another embodiment of the present disclosure, and FIG. 8 is a schematic diagram of the implant when implanted between the left atrium and the coronary sinus. The implant includes a main body 100, a first clamping member group 200f, and a second clamping member group 200f'. The first clamping member group 200f and the second clamping member group 200f' are located at different axial positions relative to the main body 100. That is, the two clamping members within the same clamping member group are different in shape, and the clamping members in different clamping member groups are also different in shape. In clamping member group 200f, the first clamping portion and the second clamping portion are offset toward one end of the main body 100, while in clamping member group 200f', the first clamping portion and the second clamping portion are offset toward the other end of the main body 100. The orientation of the clamping space defined by the multiple clamping member groups (shown by the dashed line in the figure) is non-orthogonal to the main body 100. This allows the implant to have a certain deflection angle when placed at the target part. For example, it can be implanted between the left atrium and the coronary sinus, where the space of a side near the coronary sinus is relatively small. This deflected placement manner causes one end of the main body 100 to be obliquely positioned within the coronary sinus, thereby reducing the occupied space.

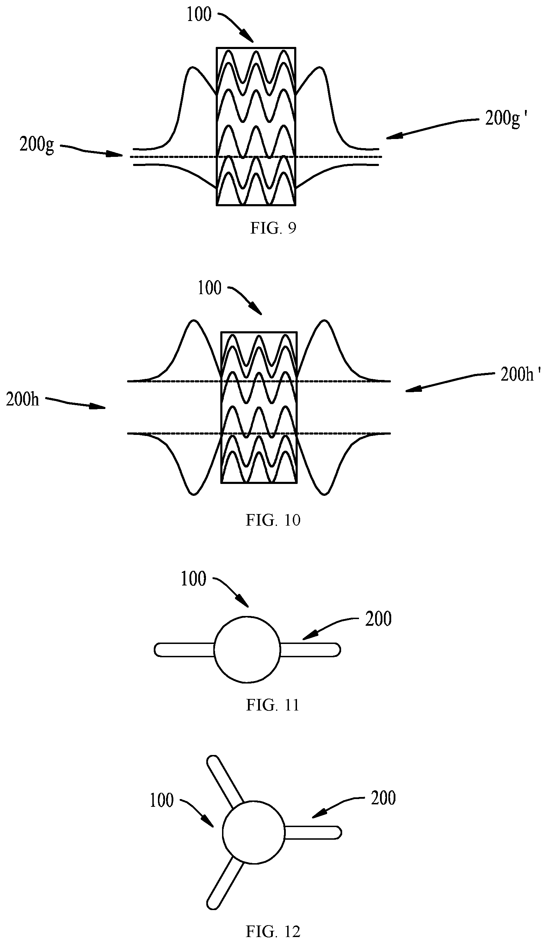

Referring to FIG. 9, FIG. 9 is a schematic structural diagram of an implant according to another embodiment of the present disclosure. The implant includes a main body 100, a first clamping member group 200g, and a second clamping member group 200g'. Within the same clamping member group, the first clamping member and the second clamping member are different in shape, causing the first clamping portion and the second clamping portion to be offset toward one end of the main body. The first clamping member group 200g and the second clamping member group 200g' have identical structures and are uniformly distributed along the circumferential direction of the main body 100. This configuration is also suitable for implantation between the left atrium and the coronary sinus, as shown in FIG. 9.

Referring to FIG. 10, FIG. 10 is a schematic structural diagram of an implant according to another embodiment of the present disclosure. The implant includes a main body 100, a first clamping member group 200h, and a second clamping member group 200h'. In this embodiment, the spacing between the two clamping members in the first clamping member group 200h and the second clamping member group 200h' is relatively large, allowing adaptation to thicker tissues at the implantation part, such as double-layered blood vessels (arterial and venous walls) in arteriovenous fistula surgery.

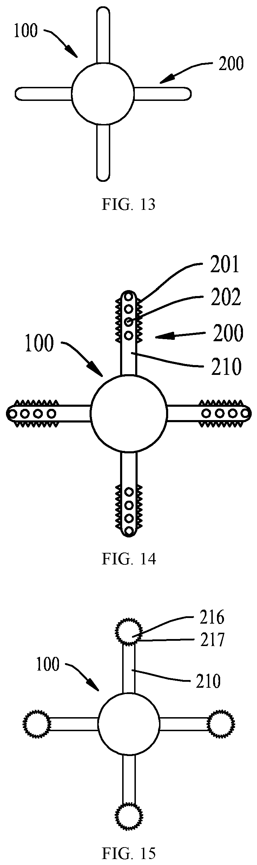

Referring to FIG. 11 to FIG. 14, implants with different numbers of clamping member groups are illustrated. The implant shown in FIG. 11 has two clamping member groups, distributed 180 degrees apart along the circumferential direction of the main body 100. The implant shown in FIG. 12 has three clamping member groups, distributed 120 degrees apart along the circumferential direction of the main body 100. The implants shown in FIG. 13 and FIG. 14 have four clamping member groups, distributed 90 degrees apart along the circumferential direction of the main body 100. FIG. 11 to FIG. 14 are top views, meaning structural diagrams viewed along the axial direction of the main body 100. In the above embodiments, the two clamping members of each clamping member group are at the same circumferential position on the main body 100, so in the top view, the two clamping members within the same clamping member group appear overlapped.

In the embodiments illustrated in FIGS. 11 to 14, the clamping members may be U-shaped structures formed by bending elastic wires, with their ends shaped as arcs. Alternatively, the clamping members may be U-shaped structures cut from metal sheets. Furthermore, when viewed from the radial direction of the main body 100, the clamping members may also adopt the structures shown in FIGS. 2 to 7.

The implant shown in FIG. 14 is structurally similar to the one shown in FIG. 13, with the difference being that the clamping members are additionally provided with anti-skid structures. For example, the end (clamping portion) of the first clamping member 210 is provided with a plurality of anti-skid protrusions 201 and anti-skid holes 202. Both features enhance the static friction between the clamping members and the tissue, enabling the implant to be more securely fixed at the target part. In some optional embodiments, only anti-skid protrusions 201 or only anti-skid holes 202 may be provided.

Referring to FIG. 15, FIG. 15 is a schematic structural diagram of an implant according to another embodiment of the present disclosure. The implant includes a main body 100 and four clamping member groups. The clamping members in the clamping member groups are similar in structure to those in FIG. 14, with the difference being the structure of the clamping member ends in this embodiment. Taking the first clamping member 210 as an example, its main body is formed by bending an elastic wire, and its end is provided with a ring 216. The ring 216 is further wrapped with a radiopaque material 217. The ring 216 prevents the end of the clamping member from puncturing the tissue. The radiopaque material can provide imaging reference information to the physician during the implantation process.

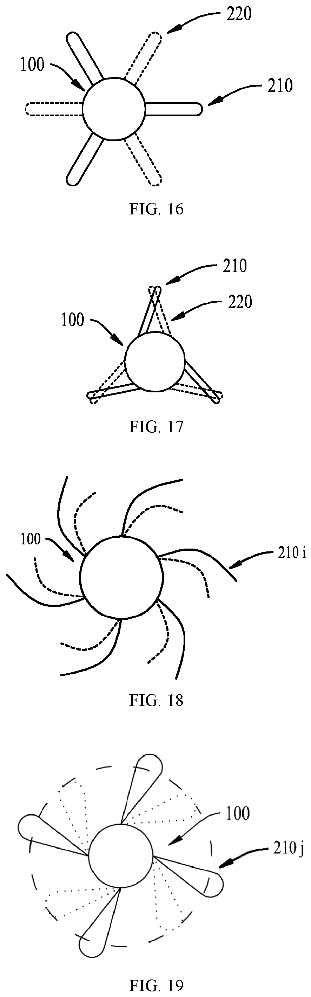

Referring to FIGS. 16 and 17, top views are shown where the clamping members within the same clamping member group are positioned differently along the circumferential direction. The implant shown in FIG. 16 includes three clamping member groups. Within the same group, the first clamping member 210 and the second clamping member 220 are completely offset along the circumferential direction, meaning they do not overlap at all when viewed axially. In this embodiment, the first clamping member 210 and the second clamping member 220 are arranged radially from the main body 100. The circumferential angular spacing between the first clamping member 210 and the second clamping member 220 is 60 degrees. In the implant shown in FIG. 17, the first clamping member 210 and the second clamping member 220 within the same group are partially offset along the circumferential direction, meaning some parts of their structures overlap when viewed axially. Preferably, their starting ends are offset circumferentially, while their end portions partially overlap. The first clamping member 210 and the second clamping member 220 are partially or completely offset circumferentially. It can increase the projected area, improve fixation effectiveness, and reduce tissue damage.

FIGS. 18 to 23 illustrate implants with clamping members that can deflect along the circumferential direction.

Referring to FIG. 18, FIG. 18 is a schematic structural diagram of an implant according to another embodiment of the present disclosure, viewed along the axial direction. In this embodiment, the implant includes a main body 100 and six clamping member groups uniformly distributed in the circumferential surface of the main body 100. Taking the first clamping member 210i as an example, it may be a structure formed by bending an elastic wire. The first clamping member 210i extends outward along the radial direction, and its latter segment curves along the circumferential direction, causing it to deflect circumferentially relative to the main body 100. In this way, when the implant is placed at a target part, if there is abundant surrounding tissue, the surrounding tissue can compress the first clamping member 210i, causing it to move closer to the surface of the main body 100 (as shown by the dashed line in the figure). This allows the clamping member to retract toward the center, thereby reducing its diameter. This can reduce damage to the surrounding tissue. When viewed from the radial direction of the main body 100, the first clamping member 210i may also adopt the curved or wound structures shown in FIGS. 2 to 7. This enables the clamping member to achieve radial contraction both through circumferential deflection and by bending toward the main body along the radial direction. In some optional embodiments, when viewed from the axial direction of the main body 100, the end of the first clamping member 210i may also be a wound structure, thereby forming a smooth structure at its outermost to prevent puncture injury to tissue.

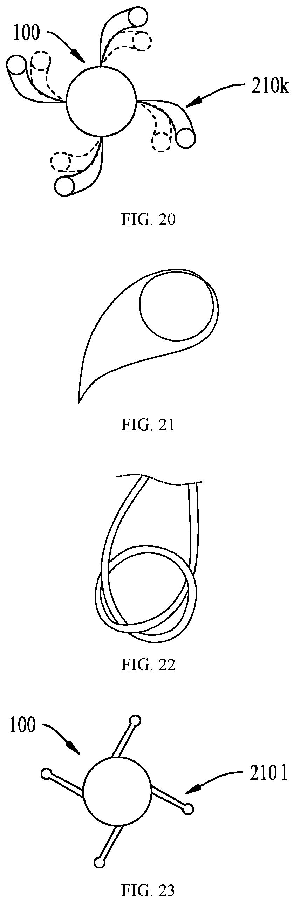

Referring to FIG. 19, FIG. 19 is a schematic structural diagram of an implant according to another embodiment of the present disclosure. Compared to the implant structure shown in FIG. 20, in this embodiment, each clamping member is offset by a certain angle along the circumferential direction relative to the main body 100. Specifically, when viewed from the axial direction of the main body 100, the first clamping member 210j is a teardrop-shaped structure formed by bending a metal wire or a curved elongated sheet structure formed by cutting a metal sheet. The front segment (the part connected to the main body 100) of the first clamping member 210j is a connecting segment, and the latter segment (the free-end side) is a clamping segment. The circumferential width of the connecting segment of the first clamping member 210j is less than the circumferential width of the clamping segment and/or the bend portion. That is, when viewed from the axial direction, the width of the connecting segment is less than that of the clamping segment. Specifically, when viewed from the axial direction, the first clamping member 210j has a shape with a gradually varying width. The base of the first clamping member 210j (i.e., the starting end of the connecting segment, which starts from an end portion on a surface of the main body 100 and is connected to the main body 100) has a smaller width, and the width gradually increases as the first clamping member 210j extends toward a distal end, such that the distal end has the largest width. Preferably, in the original state, there is an angle between the first clamping member 210j and the diameter of the main body passing through its base, and this angle is less than 90 degrees. In this way, when the first clamping member 210j is implanted at the target part, if there is abundant surrounding tissue, the surrounding tissue can easily drive the first clamping member 210j to pivot around its base, moving it closer to the surface of the main body 100. This causes the first clamping member 210j to retract toward the center, reducing its diameter. Additionally, the base of the first clamping member 210j has a smaller width and lower strength compared to end, thereby enabling easier adaptive deflection.

Referring to FIGS. 20 and 21, FIG. 20 is a schematic structural diagram of an implant according to another embodiment of the present disclosure, and FIG. 21 is a schematic diagram of the winding manner of the clamping member in FIG. 20. In this embodiment, when viewed from the axial direction of the main body 100, the first clamping member 210k is a teardrop-shaped structure formed by bending a metal wire or a curved elongated structure formed by cutting a metal sheet. The base of the first clamping member 210k (starting from an end portion on the surface of the main body 100 and connected to the main body 100) has a smaller width, while the distal end has a larger width.. The first clamping member 210k curves circumferentially, allowing it to subject a greater circumferential component of force when compressed by surrounding tissue, facilitating its adaptive retraction. In this embodiment, the end of the first clamping member 210k is a ring structure, which can enhance the clamping performance of the first clamping member 210k on tissue. The ring structure may also be wrapped with radiopaque material. Preferably, the ring structure is integrally formed with the rest of the first clamping member 210k, both wound from the same metal wire. During the winding process, the metal wire extends unidirectionally along the axial direction of the ring structure, meaning adjacent parts of the wire within the ring structure do not interlace. The winding manner of the clamping member shown in FIG. 21 is merely schematic, and the specific details thereof are not limited to those illustrated in the figure.

FIG. 22 is a schematic diagram of the winding manner for a clamping member in an alternative implementation. Compared to the winding manner shown in FIG. 21, the metal wire interlaces during the winding process. That is, the metal wire reciprocates along the axial direction of the ring structure during winding, and adjacent parts of the wire within the ring structure intertlace. This manner facilitates the winding of radiopaque material and can enhance imaging visibility.

Referring to FIG. 23, FIG. 23 is a schematic structural diagram of an implant according to another embodiment of the present disclosure. In this embodiment, when viewed from the axial direction of the main body 100, the shape of the first clamping member 210l is generally U-shaped, consisting of two rod segments and a ring with a diameter larger than the spacing between the two rod ends. The ring is located at the free end of the first clamping member 210l. The first clamping member 210l extends radially from the main body 100 and forms an angle with the diameter passing through the base of the first clamping member 210l. The end of the first clamping member 2101 is a ring structure, which can be wrapped with radiopaque material.

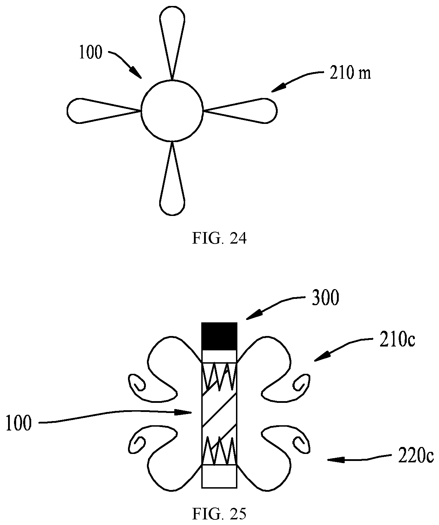

Referring to FIG. 24, FIG. 24 is a schematic structural diagram of an implant according to another embodiment of the present disclosure. The implant in this embodiment is substantially similar in structure to the implant shown in FIG. 19, with the difference being that the first clamping member 210m does not deflect or deform in the circumferential direction of the main body 100 but is arranged in a standard centrally radial pattern. The base of the first clamping member 210m has a smaller width and lower strength. Therefore, if it encounters compression from surrounding tissue during implantation, the main body 100 can be manually rotated to cause the first clamping member 210m to deflect relative to the main body 100.

FIGS. 18 to 24 all use the first clamping member for illustration. It is understandable that, when viewed the axial direction, the second clamping member may have the same shape as the first clamping member 210j or a different shape, such as deflecting in the opposite direction. When the first clamping member and the second clamping member deflect, the overall radial dimension of the implant is reduced, thereby avoiding tissue damage.

The implants shown in FIGS. 18 to 24 feature clamping members that can deflect along the circumferential direction. While the entire clamping member deflects along the circumferential direction, the distance between its outermost side/outermost end and the centerline of the main body 100 also decreases. This effectively achieves radial contraction through circumferential deflection. In some optional embodiments, the latter part of the clamping member may also bend circumferentially relative to the former part, creating local circumferential deflection and thus achieving radial contraction. As seen with the first clamping member 210i in FIG. 18, its shape in the axial view is a broken line, and the latter segment of the line can bend relative to the former segment, forming local circumferential deflection.

Referring to FIG. 25, FIG. 25 is a schematic structural diagram of an implant according to another embodiment of the present disclosure. In this embodiment, the implant is a sensing device. The main body 100 includes a sensor 300 and a connecting member. The connecting member is, for example, a heat-shrink tube. The sensor 300 is fixed inside the connecting member, and one end of the clamping member is located between the connecting member and the sensor, coaxially connected via a heat-shrinking process. The sensor is maintained at the target part through the connecting member and the clamping member.

FIGS. 1b to 11 primarily introduce the design of the implant from a radial perspective, while FIGS. 12 to 24 mainly focus on the axial perspective design. It is understandable that any radial perspective design can be combined with any axial perspective design. Therefore, one or more of the above embodiments can be combined to form new embodiments. Although the main body 100 is illustrated as cylindrical in the accompanying drawings, those skilled in the art will understand that the main body 100 may also take other tubular shapes, such as a truncated cone shape. The present invention does not impose limitations in this regard.

The above descriptions are merely specific embodiments of the present disclosure. However, the scope of protection of the present disclosure is not limited thereto. Any modifications, substitutions, or combinations that can be readily conceived by a person skilled in the art within the technical scope disclosed by the present disclosure should be encompassed within the protection scope of the present disclosure. Therefore, the protection scope of the present disclosure shall be defined by the scope of the appended claims.

## Claims

1. An implant, comprising:
a main body, configured to be fixed to a target part, and the main body having a predetermined length; and
at least one clamping member group, the clamping member group comprising a first clamping member and a second clamping member, and the first clamping member and the second clamping member being distributed on a periphery of the main body at an interval along the axial direction of the main body;
wherein the first clamping member and the second clamping member are configured to be elastically deformable.

2. The implant according to claim 1, wherein the implant is used for improving the physiological function and/or detecting physiological indicators of the target part; the first clamping member and the second clamping member are configured to be capable of contracting along the radial direction of the main body and/or moving away from each other along the axial direction of the main body through elastic deformation.

3. The implant according to claim 1 or 2, wherein the first clamping member and/or the second clamping member comprises a connecting segment and a clamping segment, one end of the connecting segment is connected to the main body and the other end is connected to the clamping segment, and the first clamping member and the second clamping member protrude entirely from the outer peripheral surface of the main body.

4. The implant according to claim 3, wherein the connecting segment is configured to be deformable so as to move away from each other along the axial direction of the main body.

5. The implant according to claim 3 or 4, wherein the clamping segment is configured to be deformable so as to contract along the radial direction of the main body.

6. The implant according to any one of claims 3 to 5, wherein the connecting segment is configured to be deformable so as to contract along the radial direction of the main body.

7. The implant according to any one of claims 3 to 6, wherein the clamping segment is configured to be deformable so as to move away from each other along the axial direction of the main body.

8. The implant according to claim 5, wherein the clamping segment deforms in a form of circumferential deflection so as to contract along the radial direction of the main body.

9. The implant according to claim 5, wherein the clamping segment deforms in a form of bending radially toward the main body so as to contract along the radial direction of the main body.

10. The implant according to claim 6, wherein the connecting segment deforms in a form of circumferential deflection so as to contract along the radial direction of the main body.

11. The implant according to claim 6, wherein the connecting segment deforms in a form of bending radially toward the main body so as to contract along the radial direction of the main body.

12. The implant according to any one of claims 3 to 10, wherein a bend portion with a variable angle is provided between the clamping segment and the connecting segment, so that the clamping segment is capable of being compressed along the radial direction of the main body.

13. The implant according to any one of claims 3 to 10, wherein the clamping segment is configured such that at least a part thereof is capable of changing the angle between the clamping segment and the connecting segment in a form of circumferential deflection and/or bending toward the main body, so that the clamping segment is capable of being compressed along the radial direction of the main body.

14. The implant according to any one of claims 1 to 13, wherein the outermost end of the clamping segment is a smooth structure.

15. The implant according to claim 14, wherein the clamping segment is formed by bending or winding an elastic wire or an elastic sheet, and the end of the elastic wire or the elastic sheet is located between the outermost end and the main body.

16. The implant according to claim 14, wherein the clamping segment is formed by cutting an elastic sheet, and the end of the elastic sheet is arc-shaped.

17. The implant according to claim 15 or 16, wherein when the clamping segment is formed by winding the elastic wire or the elastic sheet, the end of the elastic wire or the elastic sheet is located inside a wound structure.

18. The implant according to claim 17, wherein the elastic wire or the elastic sheet is entirely wound or the end of the elastic wire or the elastic sheet is wound, so that the outermost end of the clamping segment is a smooth structure.

19. The implant according to any one of claims 1 to 18, wherein the first clamping member and the second clamping member further bend circumferentially to be capable of deflecting circumferentially relative to the main body under an external force, the first clamping member and the second clamping member form an angle with the main body, and the angle is less than 90 degrees.

20. The implant according to any one of claims 1 to 19, wherein the circumferential width of the connecting segment of the first clamping member and the second clamping member is less than the width of the clamping segment and/or the width of the bend portion, so as to facilitate the first clamping member and the second clamping member to deflect circumferentially relative to the main body under an external force.

21. The implant according to any one of claims 14 to 20, wherein the outermost end is a ring structure, and the ring structure is provided with a radiopaque material.

22. The implant according to any one of claims 3 to 13, wherein the clamping segment comprises a clamping portion, and the clamping portion of the first clamping member and the clamping portion of the second clamping member have a minimum spacing between the first clamping member and the second clamping member along the axial direction of the main body.

23. The implant according to any one of claims 3 to 13, wherein the clamping portion of the first clamping member and the clamping portion of the second clamping member are parallel to each other.

24. The implant according to claim 22 or 23, wherein the clamping portion is a ring structure in an axial or radial view of the main body.

25. The implant according to claim 24, wherein the ring structure is formed by winding a metal wire.

26. The implant according to claim 25, wherein the metal wire is wound in an interlaced form in the ring structure.

27. The implant according to claim 22 or 23, wherein the number of the clamping member groups is multiple, and the clamping portions of at least two of the clamping member groups are located at different axial positions relative to the main body.

28. The implant according to claim 22 or 23, wherein the first clamping member and the second clamping member are different in shape, so that the clamping portion is offset to one end of the main body.

29. The implant according to any one of claims 1 to 13, wherein the first clamping member and the second clamping member are partially offset or completely offset along the circumferential direction.

30. The implant according to claim 29, wherein the first clamping member and the second clamping member are partially offset along the circumferential direction, with their starting ends offset circumferentially and their ends coinciding.

31. The implant according to any one of claims 3 to 13, wherein the clamping segment comprises a clamping portion, and the clamping portion is provided with anti-skid protrusions or anti-skid holes.

32. The implant according to any one of claims 1 to 31, wherein the main body comprises a cylinder, the cylinder is formed by spirally winding an elastic curved segment made of metal material, and the first clamping member and the second clamping member are integrally formed with the cylinder.

33. The implant according to any one of claims 1 to 31, wherein the main body comprises:
a cylinder of a shunt device.

34. The implant according to any one of claims 1 to 31, wherein the main body comprises: a sensor and a connecting member, and the connecting member is connected to the clamping member group.
